Europäisches Patentamt

European Patent Office

Office européen des brevets

(11) Publication number: **0 133 540**
A1

## (12) EUROPEAN PATENT APPLICATION

(21) Application number: **84109091.3**

(22) Date of filing: **01.08.84**

(51) Int. Cl.⁴: **G 01 N 33/577**
**G 01 N 33/566, G 01 N 33/554**

(30) Priority: **03.08.83 US 519992**

(43) Date of publication of application:
**27.02.85 Bulletin 85/9**

(84) Designated Contracting States:
**AT BE CH DE FR GB IT LI LU NL SE**

(71) Applicant: **E.I. DU PONT DE NEMOURS AND COMPANY**
**1007 Market Street**
**Wilmington Delaware 19898(US)**

(72) Inventor: **Reilly, Thomas Michael**
**26 Wayne Drive**
**Wilmington Delaware 19809(US)**

(74) Representative: **von Kreisler, Alek, Dipl.-Chem. et al,**
**Deichmannhaus am Hauptbahnhof**
**D-5000 Köln 1(DE)**

(54) Receptor assays using labeled monoclonal anti-idiotypic antibodies.

(57) Labeled, monoclonal, anti-idiotypic antibodies capable of binding to preselected biospecific cellular receptors are contacted with peripheral blood cells. The amount of label biospecifically adsorbed to the cells is a measure of the receptor level on the cell. This receptor level measurement can be used as an alternative to tissue biopsy.

EP 0 133 540 A1

1

## TITLE

## RECEPTOR ASSAYS USING LABELED MONOCLONAL ANTI-IDIOTYPIC ANTIBODIES

### Technical Field

This invention relates to assay methods for the quantitation of cellular receptors on peripheral blood cells and, more particularly, to the use of monoclonal labeled anti-idiotypic antibodies as ligand mimics for the quantitation of receptors.

### Background Art

The physiological effects of many biological effector molecules, such as hormones, drugs, neuropeptides, neurotransmitters, and growth factors, are mediated through their interactions with biospecific receptors on their target cells. These receptors may be broadly subdivided into two categories: (1) intracellular receptors, present in the cytoplasm or nucleus of the cell; and (2) surface receptors, present in the cell membrane. Although the two classes of receptors differ chemically, the principles of selective and specific ligand binding apply equally to both.

In man, a number of pathological states are characterized by alterations in the properties of biospecific receptors, the most prevalent of these alterations being a change in apparent receptor level. Changes in apparent receptor level can arise from appearance or loss, change in numbers, binding constants, availability, etc. It is therefore often of interest to be able to quantitate the apparent number of receptors for a given ligand in a given tissue. In some cases, receptors can occur on peripheral blood cells (lymphocytes, erythrocytes, platelets, etc.) where they can be assayed without the need for biopsy. Table I lists a number of such receptors, the cell types on which they can be found and the disease states in which their apparent level is altered.

Currently available methods of quantitating receptors are time-consuming and labor-intensive. Typically, the tissue of interest is first biopsied, then homogenized, and then incubated with an appropriate radiolabeled ligand until equilibrium is reached. The homogenate is then washed exhaustively to remove any unbound label and the amount of bound label is quantitated by scintillation or gamma counting. Not only is this procedure cumbersome, but it is frequently difficult to radiolabel small ligands without a resulting loss of activity. Furthermore, the use of radiolabels poses numerous hazards with respect to their storage, handling, and disposal. The short half-life of such commonly-used radioisotopes as $^{125}I$ and $^{131}I$ also makes them unattractive for routine clinical laboratory use. Finally, the propensity of small hydrophobic ligands to bind nonspecifically to sites on the cell membrane, that is, to non-receptor sites, often leads to erroneous values when radiolabeled ligands are used to measure receptors [Dax et al., Molecular Pharmacology, Volume 22, 5 (1982)].

Receptor immunoassays, utilizing specific anti-receptor antibodies, provide an attractive alternative to the traditional method described above. Anti-receptor antibodies can be labeled conveniently with isotopic or non-isotopic labels, for example, enzymes and fluorophores. However, the production of antibodies having the desired properties (specificity, high affinity, etc.) has been limited by the difficulty of obtaining receptors in sufficiently pure form to render them useful as immunogens. Receptors typically occur in extremely low concentrations, typically less than 0.01% of the total membrane protein, and are difficult to purify. Most of the presently available anti-receptor antibodies have been raised against receptors isolated from non-human sources. Typically, these antibodies recognize framework determinants rather than the ligand binding site and often do not cross-react with the equivalent receptor from human tissue [Strosberg et al., Immunol. Today, Volume 4, p. 75 (1981)]. Because of these practical difficulties, receptor immunoassays have not found widespread clinical use.

It is possible to prepare anti-receptor antibodies which mimic the ligand in its binding behavior vis a vis the receptor by preparing antibodies to idiotypic determinants on an anti-ligand antibody. The term idiotype or idiotypic determinant refers to the antigenic determinant(s) of an immunoglobulin (antibody) molecule which is (are) associated with the Fv region. Antibodies are composed of two heavy (H) chains and two light (L) chains, associated via one or more disulfide bonds. Each chain can be subdivided into a C-terminal constant region and an N-terminal variable (V) region. The $V_H$ and $V_L$ regions taken together make up the Fv region of an immunoglobulin. Antibodies which react with determinants in the Fv region are referred to as anti-idiotypic antibodies.

Many idiotypic determinants are located in or very close to the ligand-binding site. In such cases, ligand can competitively inhibit the binding of anti-idiotypic antibody to idiotypic antibody (anti-ligand antibody). Such anti-idiotypic antibodies are said to be ligand-inhibitable, and they behave as ligand mimics.

Sege et al., Proc. Natl. Acad. Sci. (USA), Volume 75, 2443 (1978), describe the production of polyclonal anti-idiotypic antibodies to anti-insulin which bind to the insulin receptor in an insulin-like fashion.

Schreibel et al., Proc. Natl. Acad. Sci. (USA), Volume 77, 7385 (1980), raised polyclonal anti-idiotypic antibodies to anti-alprenolol, alprenolol being a ß-adrenergic receptor antagonist. These anti-idiotypic antibodies bound to the ß-adrenergic receptor and modulated the catecholamine-sensitive enzyme adenylate cyclase.

Other investigators have raised anti-idiotypic antibodies to anti-ligand antibodies which react with the formyl peptide receptor [Maresco et al., J. Immunol., Volume 128, 963 (1982)] and the acetylcholine receptor [Wasserman et al., Proc. Natl. Acad. Sci. (USA), Volume 79, 4810 (1982)].

The antibodies described above are all polyclonal and are all too low in affinity for their respective receptors to be of any practical use in a diagnostic immunoassay.

Kohler and Milstein, Nature, Volume 256, 495 (1975), were the first to describe methods of making monoclonal antibodies by fusing spleen cells from an immunized mouse to a drug-resistant plasmacytoma cell line and isolating the hybrid clones by growth on selective medium. Subsequently, there have been numerous publications in both the patent and non-patent literature of the production of monoclonal antibodies to various antigens and haptens; see, for example, Current Topics in Microbiology and Immunology: Lymphocyte Hybridomas, F. Melchers et al., ed., New York: Springer Verlag (1978), and references contained therein.

Monoclonal anti-idiotypic antibodies have also been described in the literature. [Estess et al., Progress in Immunology IV, M. Fougereau, ed., New York: Academic Press (1980)]. However, there have been no reports of monoclonal anti-idiotypic antibodies raised to anti-ligand antibodies. The clinical
utility of receptor assays will increase with the
availability of rapid and sensitive assay methods
which do not require biopsy specimens, the disadvantages of which are self-evident. As more receptor
assays are performed, more correlations of apparent
receptor level on peripheral blood cells with various
disease states will emerge.

There is a need for receptor assays which do not
require radiolabeled ligand; do not require purified
receptor; do not require biopsy specimens; and are
rapid, sensitive, and potentially amenable to automation.

## Disclosure of the Invention

The instant invention provides an improvement
over existing methods for the assay of receptors on
normally biopsied tissues. These existing methods
comprise incubating tissues with radiolabelled biospecific receptor ligands. The improvement comprises:

incubating a peripheral blood cell
having at least one biospecific receptor
present at an apparent level which can be
correlated with a (i) specific disease
state, (ii) choice and efficacy of therapeutic regimen or (iii) physiological
state of the cell with a monoclonal anti-
idiotypic antibody which is directly or
indirectly labeled and which is reactive
with the receptor; and

measuring the amount of the label
which is biospecifically adsorbed on said
cell; and

correlating the amount of measured
label with a reference value.

The amount of label bound to the cell is proportional to the apparent receptor level on the cell. The label can be an enzyme, fluorophore, chemiluminescent material, bioluminiscent material, enzyme inhibitor, cofactor, as well as a radioisotope. Numerous other labeling substances are known in the patent and non-patent literature. The antibody can be directly labeled or can be indirectly labeled by use of a directly labeled heterologous antibody reactive with the $F_c$ region of the anti-idiotypic antibody. In addition, the anti-idiotypic antibody can be coupled to one of two complementary molecules such as biotin or avidin and a conventional label (radioisotope, enzyme, fluorophore, etc.) can be coupled to the other molecule (avidin or biotin, respectively). The interaction of the two complementary molecules will link indirectly the conventional label to the anti-idiotypic antibody.

## Description of the Invention

The production of antibodies to ligands is well-known in the art. Antibodies can be prepared either by conventional immunization or by cell fusion techniques, the latter method yielding monoclonal or hybridoma antibodies. Typically, an animal is immunized with the ligand of interest emulsified in adjuvant and boosted at regular intervals. The serum is assayed for the presence of anti-ligand antibody by any convenient method, frequently RIA or ELISA. Once an acceptable titer of antibody is found, the animal can be bled and the antibodies purified from serum, or the animal (usually a mouse) sacrificed and its spleen removed aseptically for fusion. In the latter case, a single cell suspension is prepared and the immune spleen cells are fused using polyethylene glycol (PEG) with a hypoxanthine guanine phosphoribosyl transferase (HGPRT)-deficient plasmacytoma line. Several such lines are known (See Current Topics in Microbiology and Immunology, cited above), of which the most preferred are those lines which do not secrete any immunoglobulin of their own. One such line is the F/O plasmacytoma line, described by Fazekas de St. Groth, et al., J. Immunol. Meth., Volume 35, 1, (1980). The fusion mixture is then plated out on selective medium containing hypoxanthine, aminopterin, and thymidine (HAT). When sufficient cell growth has occurred, the culture supernatant fluid is sampled and tested for antibody by any convenient means, frequently RIA or ELISA. Those cultures which contain antibody of interest are then cloned by limiting dilution or in soft agar, re-tested, and expanded. The clones of interest are then injected into syngeneic or congenic mice. If injected intravenously or subcutaneously, antibodies are then harvested from the serum. If injected intraperitoneally into pristane-primed animals, antibodies are harvested from ascites fluid.

Regardless of whether the anti-ligand antibody (hereinafter referred to as $Ab_1$) is produced by conventional or monoclonal techniques, it is usually desirable to purify it at least partially before producing anti-idiotypic antibodies (hereinafter referred to as $Ab_2$). Antibody purification techniques are well-known in the art. In general, the serum or ascites is precipitated with ammonium sulfate to yield an immunoglobulin (Ig) fraction and the resultant precipitate re-suspended in an aqueous medium for ion-exchange or gel filtration chromatography for isolation of the antibody class of interest. Chromatography on staphlyococcal protein A can also be used if the antibody activity resides in a protein A-binding subclass of IgG. Further purification can be achieved by chromatography on a ligand affinity column.

8

After purification, $Ab_1$ is injected into animals to produce $Ab_2$. Since it is desirable that $Ab_2$ have an affinity of at least $10^9$ $Mol^{-1}$ and be ligand-inhibitable, it is preferred that $Ab_2$ be a monoclonal antibody. Although in theory $Ab_2$ can be produced in any species, it is easier from a practical point of view to produce $Ab_2$ if $Ab_2$ is made in the same species used to make $Ab_1$. While rat x rat, rat x mouse, human x human, and mouse x human hybridomas are known, they are in general more difficult to make and less stable than mouse x mouse hybridomas. Therefore, it is generally preferred that $Ab_2$ be made in a mouse and even more preferred that it be made in a syngeneic mouse to minimize the production of antibodies to non-idiotypic determinants.

$Ab_2$ is screened for its ability to bind to receptor in a ligand-inhibitable manner. Once a suitable antibody has been identified, it is purified as described above and labeled.

$Ab_2$ can be further screened for its ability to bind to the receptor with an association constant of at least about $10^9$ $Mol^{-1}$ by Scatchard analysis.

Numerous labels are known in the art. Among the most widely used are enzymes such as ß-galactosidase, horseradish peroxidase, and alkaline phosphatase. Where the label is an enzyme, it is desirable that it have a high turnover number, a readily available chromogenic or fluorogenic substrate, and appropriate functional groups for coupling to $Ab_2$. In addition, it is useful if the enzyme is stable and not present in human serum. Suitable functional groups on the enzyme for coupling include amino, carboxyl, and sulfhydryl groups and carbohydrate moieties. The same functional groups can also be utilized on $Ab_2$.

In general, it is desirable to label $Ab_2$ as extensively as possible without loss of either antigen-binding or enzymatic activity. It is usually possible to attach as many as four horseradish peroxidase molecules per antibody, but probably only one ß-galactosidase molecule because of its several-fold higher molecular weight.

Numerous coupling techniques are known in the art. The specific technique chosen depends on the functional groups available on the enzyme and the antibody. For example, ß-galactosidase has a free sulfhydryl group and horseradish peroxidase has carbohydrate moieties which can be utilized for coupling.

Following labeling, it is usually desirable to purify the conjugate to remove unconjugated enzyme and antibody, as well as non-immunoreactive antibody. This can be accomplished by a combination of gel filtration and affinity chromatography steps.

For labeling purposes, $Ab_2$ can be either divalent or monovalent. Techniques for the production of monovalent antibody fragments are well-known in the art. For example, Fab fragments can be made by cleaving IgG with papain and Fab' fragments can be made by the reduction and alkylation of $F(ab')_2$ fragments, which in turn are made by pepsin clevage of IgG.

It is also possible to use unlabeled $Ab_2$, followed by a labeled antibody which is reactive with mouse immunoglobulin. Such a sandwich configuration offers the advantage that only a single labeled antibody needs to be prepared regardless of which receptor one wishes to measure. Again, the anti-mouse Ig antibody can be labeled with any of the labeling substances and by any of the methods described above. It can be employed as divalent or monovalent antibody.

0133540

Peripheral blood cells can be isolated by known methods. The most commonly-employed method is probably density gradient centrifugation. For example, red blood cells can be separated from lymphocytes and platelets on a Ficoll-Hypaque gradient. Bovine serum albumin and colloidal silica gradients can also be employed. Platelets can be separated from lymphocytes by low speed centrifugation. Lymphocytes can be fractionated into B and T cells on Percoll gradients.

The assay of this invention can be used to quantify cellular receptors which occur either on the cell surface or in the cytoplasm. To measure cytoplasmic receptors, it is first necessary to lyse the cells. Lysis with a non-ionic detergent such as Triton X-100 or Nonidet P-40 is usually preferred, but osmotic lysis is also possible.

The assay of this invention for cellular associated receptors is carried out by incubating isolated peripheral blood cells with a labeled monoclonal anti-idiotypic antibody; washing the cells to remove unbound labeled antibody; and measuring the amount of label bound to the cell surface. The peripheral blood cells can be erythrocytes, lymphocytes, granulocytes, or platelets and can be isolated by any suitable method, most commonly density gradient centrifugation. The cells are washed with a buffered isotonic TMR solution or with medium. They are then counted either manually on a hemocytometer or automatically using, for example, a Coulter counter, and adjusted to the desired concentration. If necessary, cell viability can also be determined at this stage, usually by staining with a vital dye. They are then incubated for a variable length of time with enzyme-labeled monoclonal anti-idiotypic antibody. Incubation times will typically range from five minutes to three hours, most often from thirty minutes to one hour. The temperature of incubation can range from 0°C to 45°C, most often 37°C. The cells will usually be incubated in an isotonic solution, buffered to a pH between 4 and 10, more often between 6 and 8. The buffer can contain other substances, for example, 0.1 to 1.0% (by weight) BSA. In some cases, it is desirable to include preservatives or metabolic inhibitors for the dual purpose of preventing microbial contamination and preventing receptor modulation. It is also possible to incubate the cells in a nutrient medium; this can be especially desirable if prolonged incubation times are to be used and cell viability at the time of assay is required. The type of medium used depends on the cells being assayed. Typically, RPMI-1640 and Dulbecco's modified essential medium (DMEM) are used. Following incubation, the cells are washed with buffer or medium, usually the same buffer or medium in which the incubation was performed. Washing is usually accomplished by suspension in washing medium followed by centrifugation and aspiration of the supernatant fluid. Alternative methods are possible. For example, the cells can be washed by filtration. The washed cells are then resuspended in a suitable buffer containing any necessary reagents for the detection of label. If the label is an enzyme, a substrate and any necessary cofactors and cations for assay of the bound enzyme label will be included in the buffer. Enzyme activity can be measured photometrically or fluorimetrically as either the rate of change in absorbance (or transmission) at a suitable wavelength or as an end point determined after a fixed length of time. The temperature at which activity is determined can range from 0°C to 45°C, most often from 25°C to 37°C.

The amount of enzyme activity (if label is an enzyme) can be related to the apparent receptor level either by comparison with a standard curve or by Scatchard analysis.

When other labeling substances are used, other measurements of apparent receptor level are possible. For example, if the label is a fluorophore the receptor density can be determined by fluorescence-activated cell sorting analysis (FACS). This type of analysis offers the advantage of full automation.

When the measured receptor level is to be used as a diagnostic marker of a disease state, the receptor level on cells from a patient is compared to a reference which will be the receptor level on cells from normal individuals. Changes in receptor levels on a patient's cells, in comparison to reference values, will often be indicative of certain disease states including some of the diseases listed in Table 1. For example, a 66% decrease in the binding of glucagon to circulating mononuclear cells from patients with diabetes compared with non-diabetic controls has been reported. [Goldstein et al., Endocrine Research Communication, Volume 2, 367 (1980)]

When the measured receptor level is to be used by a clinician to design a specific therapeutic regimen for a particular patient or to monitor the efficacy of such treatment, the receptor level will be compared to a reference which will be the receptor level on cells from the same individual prior to the initiation of therapy or the receptor level on cells from normal, untreated individuals. For example, measurement of the number of lymphocyte glucocorticoid receptors in non-Hodgkins lymphoma and in acute lymphoblastic leukemia is useful in selecting those patients likely to benefit from glucocorticoid therapy. [Shipman et al., Blood, Volume 58, p. 1198 (1981)]

When the measured receptor level is to be used to monitor the physiological state of the cells, such as activation caused by mitogens, viruses or tumor-induced transformation, the receptor level will be compared to a reference which will be the receptor level on unactivated cells obtained from normal individuals. For example, resting T-lymphocytes possess few glucagon receptors. However, following activation of such cells with T-cell specific mitogens, the numbers of such receptors increases dramatically, indicating that the glucagon receptor is a marker of activated T-lymphocytes [Bhathena et al., Endocrinology, Volume 111, p. 684 (1982)]

The following example illustrates the invention.

## EXAMPLE

Preparation of monoclonal anti-idiotypic antibodies and their use in measuring receptors on blood cells is described below using as an example anti-idiotypic antibodies reactive with the glucagon receptor. Similar methodology will apply when preparing and utilizing anti-idiotypic antibodies reactive with other receptors.

## PART 1
### Preparation of Anti-Idiotypic Antibodies
### Reactive with Cellular Receptors

A. **Preparation of Anti-glucagon Antibodies (idiotype)**

Glucagon was covalently linked to the carrier keyhole limpet hemocyanin (KLH) using carbodiimide. 0.5 mg of glucagon and 2.5 mg of KLH were dissolved in 1.5 mL distilled $H_2O$ and titrated with 1 N NaOH until complete dissolution was achieved. 0.5 mL of an aqueous solution of 1-ethyl-3-(3-dimethylamino-propyl)-carbodiimide (EDAC, 3.5 mg/mL) was added and the solution incubated at room temperature for 18 hours. 2 mL of 0.05 M hydroxyamine hydrochloride was added and the solution incubated an additional 5 hours at room temperature. The resultant conjugate, having a ratio of glucagon to KLH of approximately 8-10:1, was emulsified in an equal volume of complete Freund's adjuvant (CFA) and 25 µg was injected into the footpads of Balb/c mice. Two weeks later the mice were boosted intraperitoneally with 12.5 µg of the conjugate emulsified in incomplete Freund's adjuvant (IFA). Thereafter the animals were boosted monthly and their sera assayed for the presence of antibody 7 days after each boost, as described below.

B. Detection of Antiglucagon Antibodies

Antibody to glucagon was detected using an enzyme-linked immunosorbent assay (ELISA). Glucagon was covalently coupled to bovine serum albumin (BSA) using carbodiimide, as described above. 10 µg of the glucagon/BSA conjugate (ratio of glucagon to BSA approximately 8-10:1), in 100 µL of phosphate buffered saline, pH 7.4 (PBS) was added to individual wells of a 96-well polyvinyl chloride microtiter plate and allowed to incubate for 1 hour at room temperature. The plates were flicked to remove excess conjugate solution and 50 µL of the test serum, diluted from $10^{-1}$ to $10^{-6}$ in PBS/0.1% BSA, was added to the wells. After incubating for 2 hours at room temperature, the plates were washed 3 times with PBS. 50 µL of sheep anti-mouse IgG conjugated to ß-galactosidase (Bethesda Research Laboratories) was added to each well and the plates incubated an additional 2 hours at room temperature with constant shaking. Plates were then washed 4 times with PBS and 50 µL of a 1 mg/mL solution of p-nitrophenyl-ß-D-galactoside (PNPG) containing 100 mM 2-mercaptoethanol was added to each well. The plates were incubated for 1 hour at room temperature with constant shaking, then the absorbance of the contents of each well was read at 414 nm to identify those sera possessing high titers of antiglucagon activity.

C.   Preparation of Anti-glucagon IgG

An Ig fraction was prepared from whole serum by precipitation with 45% saturated ammonium sulfate. The resultant precipitate was redissolved in $H_2O$ and concentrated by ultra-filtration, then dialyzed against PBS, pH 7.4.  A glucagon affinity column was prepared by coupling the hormone to CNBr-activated Sepharose 4B following the procedure recommended by the manufacturer (Pharmacia Fine Chemicals).  The ratio of glucagon to Sepharose 4B was 5 mg/g wet weight.  The Ig fraction obtained above was passed over the column and eluted with 0.23 M glycine buffer, pH 3.4.  2 M Tris, pH 7.6, was immediately added to the eluate fractions to neutralize the pH, and the fractions were precipitated with 50% saturated ammonium sulfate and dialyzed against PBS, pH 7.4.  The anti-glucagon activity in the various fractions was determined by ELISA, as described in (B) above.

D.   Preparation of Anti-idiotypic Antibodies

100 µg of the Ig fraction prepared above or 25 µg of the affinity-purified material was emulsified in CFA and injected into the footpads of Balb/c mice.  The animals were boosted intraperitoneally at monthly intervals, using antibody emulsified in IFA. After three months, the mice were bled and the serum tested for the presence of anti-idiotypic antibodies reactive with the glucagon receptor, as described in (E) below.

E.   Detection of Anti-idiotypic Antibodies Reactive with the Glucagon Receptor

Rat liver plasma membranes rich in glucagon receptors were prepared by mincing livers from Sprague-Dawley rats. The liver suspension was adjusted to a sucrose concentration of 43.3%; 25 mL of this suspension was placed in centrifuge tubes, overlaid with 10 mL of 42.3% sucrose, and centrifuged in an SW 28 rotor (Beckman Instruments, Spinco Division) for 2 hours at 27,000 rpm. Purified membranes floated to the top of the tube where they were recovered. Membrane mass was determined by the Lowry method. 25 µg of membrane protein, in 1 mM sodium bicarbonate, was incubated at room temperature with 50 µL of the test serum, at $10^{-1}$ to $10^{-3}$ dilutions in PBS/0.1% BSA, in a final volume of 100 µL of 100 mM Tris, pH 7.6, 10 mM disodium ethylenediamine-tetraacetate, 1 mg/mL BSA, and 0.05 mg/mL bacitracin. After 1 hour, 10 uL of [$^{125}$I] -glucagon (New England Nuclear, 218 µCi/ug) was added to each sample to a concentration of $5 \times 10^{-10}$ M. Incubation was continued for 30 minutes after which time the samples were pipetted over 25 uL of 1 mM sodium bicarbonate in a microfuge tube, and spun in a microfuge for 5 minutes at 10,000 x g. The supernatant fluids were aspirated and the pellets counted in a gamma counter. The serum from unimmunized mice served as a control. Those sera which substantially reduced the binding of labeled glucagon to the liver membrane preparation were judged to contain anti-idiotypic antibodies reactive with the glucagon receptor.

F. Preparation of Monoclonal Anti-idiotypic
   Antibodies Reactive with the Glucagon Receptor

Mice producing sera which responded positively in the assay described in (E) above were given a final boost and sacrificed 3 days later. Their spleens were removed aseptically and fused with cells of the F/O murine plasmacytoma line. Any number of murine plasmacytoma lines may be used, including SP2/O-Ag14 and

P3x63-Ag8.653, which are on deposit at the American
Type Culture Collection identified by numbers CRL-1581
and CRL-1580, respectively. The ratio of spleen cells
to plasmacytoma cells used in the fusion was 2:1,
polyethylene glycol 4000 was the fusing agent. Cells
were plated at a density of $2 \times 10^5$ spleen cells per
well in 96 well microtiter plates containing HAT
(hypoxanthine, aminopterin, thymidine) selective
medium. Cells were fed with fresh HAT medium on a
weekly basis for the first two weeks and thereafter
with HT (no aminopterin) medium. After 2 to 4 weeks,
the supernatant fluids from wells displaying cell
growth were tested for the presence of antibodies
reactive with the glucagon receptor using the assay
described in (E) above. Cells producing the antibody
were cloned by limiting dilution and expanded in culture. Antibody is isolated from culture supernatants
by precipitation with ammonium sulfate, followed by
DEAE-cellulose chromatography. Specificity is verified by the ELISA procedure described in (B) above
using the affinity-purified idiotype from (C) as
antigen. Class, subclass, isoelectric point, and
affinity are determined for each antibody. Class and
subclass are determined by immunodiffusion in an agar
gel, using commercially available class- and subclass-
specific antisera. Isoelectric point is determined by
isoelectric focusing in polyacrylamide gel. Affinity
is determined by Scatchard analysis.

<div align="center">PART 2</div>

Labeling of Monoclonal Anti-Idiotypic Antibodies
A. <u>Divalent Antibody/Horseradish Peroxidase Conjugate</u>

4 mg of horseradish peroxidase (HRP) is dissolved in 1 mL of 0.3 M sodium bicarbonate buffer, pH
8.1. 1 mL of 0.06 M $NaIO_4$ in distilled water is
added and the resultant mixture incubated for 30 minutes at room temperature. Excess NaIO$_4$ is neutralized by the addition of 1 mL of 0.16 M ethylene glycol. After 1 hour, the mixture is dialyzed exhaustively against 0.1 M sodium bicarbonate, pH 9.5.

The dialyzed solution containing activated HRP is added to 2 mg of anti-(anti-glucagon) antibody. After 3 hours incubation at room temperature, 5 mg of sodium borohydride is added, and the incubation is continued for 3 hours at 4°C. The mixture is then dialyzed exhaustively against PBS at 4°C. The conjugate solution is then fractionated on Ultrogel AcA 44 (LKB; 1.6 x 95 cm column; 4% polyacrylamide/14% agarose gel having a 200,000 Dalton exclusion limit) to separate labeled from unlabeled antibody, the former eluting in the void volume. The column fractions containing peak enzyme activity are pooled and concentrated to 2 mL by pressure filtration (PM-30 Amicon membrane), then purified by affinity chromatography on an anti-glucagon column, as follows.

The conjugate solution is passed through an anti-glucagon IgG affinity column (1 cm x 7 cm), followed by 100 mL of PBS. The affinity column is prepared by coupling affinity-purified antibody from (C) above to CNBr-activated Sepharose 4B at a ratio of 1 mg IgG per 1 mL wet gel. The bound conjugate is then eluted with 50 mL of 10 mM glucagon in PBS. This eluate represents the final reagent and is dialyzed sequentially six times against 4 L of PBS at 4°C.

B. Monovalent Antibody/ß-galactosidase Conjugate

Fab' fragments are prepared from affinity purified antibodies via pepsin digestion. Twenty mL of affinity-purified anti-(anti-glucagon) antibodies are concentrated to 2 mL on an Amicon stirred-cell apparatus (PM-30 membrane). The final protein concentration is adjusted to 10 mg/mL. The sample is dialyzed against 1000 mL of 0.1 M sodium acetate, pH 4.5, for 4 hours at 4°C. After dialysis, 20 μL of a 10 mg/mL solution of pepsin, dissolved in the same sodium acetate buffer, is added and the temperature raised to 37°C for 20 hours. After this digestion period, the sample is clarified by a brief centrifugation and then chromatographed on a Sephadex G-150 (cross-linked, beaded dextran having 150,000 dalton exclusive limit) column (1.5 cm x 90 cm) equilibrated in 0.015 M sodium phosphate (pH 7.4), 0.15 M NaCl (phosphate buffered saline). The column fractions containing the $(Fab')_2$ fragments, identified by gel electrophoresis, are pooled (19.2 mL) and then concentrated to 2.0 mL by pressure filtration (PM-30 Amicon membrane). After concentration, the $(Fab')_2$ fragments are reduced to their corresponding Fab' fragments by adding 40 μL of a 1 M dithiothreitol solution. The reduction is performed at 25°C for 90 min under argon. The Fab' fragments are then reacted with 14.8 mg of iodoacetamide at 25°C for 2 hours under argon. Reaction products are removed by three sequential dialyses against 4 liters of phosphate buffered saline at 4°C.

The Fab' fragments so produced are then reacted with a 20-fold molar excess of MBS (m-maleimidobenzoic acid N-hydroxy-succinimide ester). Eighty-five microliters of a 79 mM solution of MBS in tetrahydrofuran are added to the 2 mL solution of Fab' fragments and reacted for 1 hour at 25°C under argon. The mixture is desalted on a Sephadex G-25 (cross-linked, beaded dextran having 5000 dalton exclusion limit) column (1.5 cm x 40 cm) in phosphate buffered saline. The derivatized Fab' fragments which eluted in the void volume, are pooled and combined with 2 mL of β-galactosidase at 12 mg/mL in phosphate buffered saline at 4°C. After 16 hours, this solution is concentrated to 2 mL on a Amicon PM-30 pressure filtration stirred-
cell followed by column chromatography on Sepharose
4B-CL (cross-linked, macroporous agarose in bead form
having 1-5 x 10$^6$ dalton exclusion limit in a 1.5 cm
x 90 cm column). The Fab'-ß-galactosidase conjugate
is eluted with the free ß-galactosidase. The entire
peak of enzyme activity is pooled and subsequently
immunopurified on an anti-glucagon affinity column.
The procedure for immunopurification is as follows:
Pooled column fractions from the Sepharose 4B-CL
column are eluted through the anti-glucagon affinity
column (1.0 cm x 7.0 cm), followed by 100 mL of
phosphate buffered saline. The Fab'-ß-galactosidase
conjugate is then eluted with 50 mL of 10 mM glucagon
in phosphate buffered saline. This eluate represents
the final reagent and is dialyzed sequentially six
times against 4 L of phosphate buffered saline at 4°C.

<div align="center"><u>PART 3</u></div>

<div align="center"><u>Isolation of Peripheral Blood Cells</u></div>

Blood obtained from patients or normal volunteers by venipuncture is collected in heparin-containing tubes. Separation of the cellular components of
whole blood is accomplished by layering the blood
sample, diluted in an equal volume of balanced salt
solution (BSS), onto FICOLL-PAQUE (Pharmacia Fine
Chemicals, a copolymer of sucrose and epichlorhydrin
which has a weight average molecular weight between
300,000 and 500,000) and centrifuging at 400 x g for
30 minutes at 18-20°C. Erythrocytes are collected
from the bottom of the tube where they sediment, while
lymphocytes, monocytes and platelets, remain at the
sample/FICOLL-PAQUE interface. The cells recovered
from the interface are resuspended in BSS and centrifuged for 3 minutes at 2900 x g. The platelets are
collected from the top while the heavier monocytes and lymphocytes pellet to the bottom. The pellet is re-suspended in BSS and the monocytes are separated by adherence to plastic petri dishes for 30 minutes at 25°C. Lymphocytes do not adhere to the plastic and are pipetted off while the adherent cells (monocytes) are recovered from the petri dish by tapping the plate vigorously and rinsing with BSS. Separation of B and T lymphocytes is accomplished by layering the lymphoctye fraction over a discontinuous gradient of Percoll (Pharmacia Fine Chemicals, colloidal silica with non-dialyzable polyvinylpyrrolidone coating having a density of 1.130 gm/mL) and centrifuging for 10 minutes at 3000 rpm. B cells band at a lighter density than T cells and are collected at the interface between the densities 1.052 and 1.063 gm/mL.

## PART 4

### Determination of Glucagon Apparent Receptor Density on T-lymphocytes

$5 \times 10^6$ T-lymphocytes, isolated as described above, are suspended in 75 μL of BSS. 25 uL of a 1 mg/mL solution of ß-galactosidase labeled Fab' fragment of a monoclonal anti-idiotypic antibody reactive with the glucagon receptor, prepared as described above, hereinafter referred to as $Ab_2$, is added and the mixture is incubated for 1 hour at 37°C. The cells are pelleted by centrifugation and washed 3 times with BSS. After the last wash, the cells are resuspended in PBS, pH 7.4, containing 100 mM 2-mercaptoethanol and 3 mM PNPG. After a one-hour incubation at 37°C, the cells are again pelleted by centrifugation. The supernatant is withdrawn and the absorbance of the supernatant fluid measured at 414 nm. The absorbance is directly proportional to the amount of labeled $Ab_2$ bound to the cells. This value is compared to a standard curve relating absorbance to apparent receptor density.

The above described assay is a single point assay in that only one dilution of antibody is used. Single point assays will often be useful when comparing receptor levels on a patient's cells with a reference value, especially when there are large differences between the two values. In some instances, it is desirable to titrate the apparent receptor density using several dilutions of labeled $Ab_2$. Such a multi-point assay allows one to do a Scatchard analysis of the data. Scatchard analysis of glucagon receptors on T-lymphocytes is performed by incubating a series of tubes each containing a standard amount of labeled $Ab_2$ and $5 \times 10^6$ T-lymphocytes with a range of concentrations of unlabeled $Ab_2$ for 60 minutes at 37°C. Separation of free from bound $Ab_2$ and quantitation of cell-bound antibody is performed as described above in Part 4. For each concentration of unlabeled $Ab_2$, the bound/free ratio of the labeled $Ab_2$ is plotted as a function of the $Ab_2$ bound. The affinity of $Ab_2$ for the receptor may be calculated from the slope of the plot. The X intercept of the plot yields a value [Ro]. From the value [Ro] the number of receptor sites per cell can be derived according to the following formula:

$$\frac{\text{sites}}{\text{per cell}} = \frac{[Ro][\text{labeled } Ab_2] \times 6.022 \times 10^{23}}{\text{number of cells per liter}}$$

## PART 5

<u>Correlation of Apparent Receptor Density</u>
<u>with Disease State</u>

    The level of receptors on a patient's cells
obtained by single point assay or the affinity and
receptor numbers obtained by Scatchard analysis is
compared with reference values obtained by performing
similar measurements on cells from normal individuals.  Statistically significant differences in the
values may be indicative of a specific disease.  For
glucagon, a significant decrease in glucagon receptor
level on a patient's mononuclear cells, in comparison
to the reference, is suggestive of diabetes.

## -Table 1

Presence of Receptors on Peripheral Cells
and Correlations with Disease States

| RECEPTOR | CELL TYPE | DISEASE STATE | CHANGE RECEPTOR LEVEL |
|---|---|---|---|
| Insulin | Monocyte | Muscular Dystrophy | D |
| Insulin | Monocyte | Diabetes | D |
| Insulin | Erythrocyte | Chronic Liver Disease | I |
| Glucocorticoid | Lymphocyte | Leukemia | D |
| ß-Adrenergic | Lymphocyte | Leukemia | D |
| ß-Adrenergic | Lymphocyte | Asthma | D |
| $_2$-Adrenergic | Platelet | Depressive Disorders | I |
| Dopaminergic | Lymphocyte | Parkinson's Disease | D |
| Triiodothyronine | Lymphocyte | Thyroid Disease | D |
| Low Density Lipoprotein | Lymphocyte | Familial Hypercholesterolemia | D |
| Acetylcholine | Lymphocyte | Leukemia | D |
| Growth Hormone | Lymphocyte | Burkitts Lymphoma | D |
| Calcitonin | Lymphocyte | Burkitts Lymphoma | D |
| Histamine | Lymphocyte | Allergic Disease | Appea |
| -Adrenergic | Platelet | Thrombocytosis | I |
| Glucagon | Monocyte | * | * |
| Somatostatin | Monocyte | * | * |
| Prolactin | Lymphocyte | * | * |
| Muscarinic Cholinergic | Lymphocyte | * | * |
| Vasoactive Intestinal Peptide | Lymphocyte | * | * |
| Prostaglandin | Lymphocyte | * | * |
| Parathyroid Hormone | Lymphocyte | * | * |
| Endorphin | Lymphocyte | * | * |
| ACTH | Lymphocyte | * | * |
| Vasopressin | Platelet | * | * |

*No Correlation Recognized I=Apparent Increase D=Apparent Decrea

Claims

1. A method for determining the apparent level of a biospecific receptor on a peripheral blood cell, which level can be correlated with (i) a disease state, (ii) choice and efficacy of therapy or (iii) physiological state of said cell, said method comprising:

incubating the peripheral blood cell with a labeled monoclonal anti-idiotypic antibody which is reactive with the receptor; and

measuring the amount of the label which is biospecifically adsorbed on said cell; and

correlating the amount of measured label with a reference value.

2. The method of Claim 1 in which the peripheral blood cell is a T-lymphocyte.

3. The method of Claim 2 in which the receptor is the glucagon receptor.

4. The method of Claim 1 in which the labeled monoclonal anti-idiotypic antibody is labeled with an enzyme selected from the group consisting of ß-galactosidase and horseradish peroxidase.

5. The method of Claim 1 in which the labeled monoclonal anti-idiotypic antibody has an affinity constant of at least $10^9$ $Mol^{-1}$.

6. The method of Claim 1 wherein the antibody is indirectly labeled.

7. The method of Claim 1 wherein the antibody is of Mouse origin.

# EUROPEAN SEARCH REPORT

## DOCUMENTS CONSIDERED TO BE RELEVANT

EP 84109091.3

| Category | Citation of document with indication, where appropriate, of relevant passages | Relevant to claim | CLASSIFICATION OF THE APPLICATION (Int Cl 4) |
|---|---|---|---|
| A | US - A - 4 223 005 (TEODORESCU et al.)<br><br>* Abstract *<br><br>---- | 1,2 | G 01 N 33/577<br>G 01 N 33/566<br>G 01 N 33/554 |

TECHNICAL FIELDS
SEARCHED (Int. Cl 4)

G 01 N 33/00

The present search report has been drawn up for all claims

| Place of search | Date of completion of the search | Examiner |
|---|---|---|
| VIENNA | 08-11-1984 | SCHNASS |